# EUROPÄISCHE PATENTSCHRIFT

(11) **EP 4 489 691 B1**
(45) Veröffentlichungstag und Bekanntmachung des Hinweises auf die Patenterteilung: **06.05.2026**
(21) Anmeldenummer: 23709713.4
(22) Anmeldetag: 07.03.2023
(51) Int. Cl.: A61F 2/16, A61B 90/92, A61B 90/96, A61L 27/20

(54) **OPHTHALMOLOGISCHES IMPLANTAT MIT EINER MASCHINENLESBAREN PRODUKTKENNZEICHNUNG**
OPHTHALMOLOGICAL IMPLANT COMPRISING A MACHINE-READABLE PRODUCT IDENTIFIER
IMPLANT OPHTALMOLOGIQUE COMPRENANT UN IDENTIFIANT DE PRODUIT LISIBLE PAR MACHINE

(30) Priorität: 10.03.2022 DE 102022105640
(43) Veröffentlichungstag der Anmeldung: 15.01.2025
(73) Patentinhaber: Carl Zeiss Meditec AG, 07745 Jena (DE)
(72) Erfinder: NICOLI, Francesca, 73447 Oberkochen (DE); BADUR, Thorben, 73447 Oberkochen (DE)
(74) Vertreter: Hofstetter, Schurack & Partner
(86) Internationale Anmeldenummer: PCT/EP2023/055795
(87) Internationale Veröffentlichungsnummer: WO 2023/170094

(56) Entgegenhaltungen:
- EP-A1- 2 685 306
- EP-A1- 3 202 369
- WO-A2-2009/124838
- XIANPENG YANG ET AL: "Surface and Interface Engineering for Nanocellulosic Advanced Materials", ADVANCED MATERIALS, VCH PUBLISHERS, DE, vol. 33, no. 28, 9 September 2020 (2020-09-09), pages n/a, XP071876812, ISSN: 0935-9648, DOI: 10.1002/ADMA.202002264

## Beschreibung

### Technisches Gebiet

Die Erfindung betrifft ein ophthalmologisches Implantat mit einem Grundkörper, welcher eine vorzugsweise maschinenlesbare Produktkennzeichnung umfasst.

### Stand der Technik

Ophthalmologische Implantate wie beispielsweise Intraokularlinsen (IOLs) werden üblicherweise durch Produktkennzeichnungen auf der Verpackung gekennzeichnet. Die Produktkennzeichnung kann neben Herstellerinformationen den Typ der IOL sowie weitere relevante Informationen wie beispielsweise den Brechwert angeben. Die Versorgung des Patienten setzt somit voraus, dass die verpackte und ausgelieferte IOL den Angaben auf der Produktkennzeichnung entspricht.

Damit sich ein Anwender hierbei nicht auf die Verpackungsangaben verlassen muss, ist es weiterhin bekannt, eine Produktkennzeichnung auf dem ophthalmologischen Implantat selbst anzubringen. Diese Art der Kennzeichnung verhindert die Gefahr einer Verwechslung, falls ein ophthalmologisches Implantat falsch verpackt beziehungsweise die Verpackung falsch gekennzeichnet wurde. Zudem sind damit wichtige Informationen über das Implantat für Ärzte und Patienten auch nach der Operation zugänglich.

Die EP 3 202 369 A1 offenbart Intraokularlinsen mit Quantenpunkten, Materialien und Verfahren zur Herstellung optischer Linsen sowie Verfahren zur Verwendung derartiger Linsen. Die Linsen bieten detektierbare Markierungen, die zum Ausrichten, Erkennen und Korrigieren der Ausrichtung von Linsen vor, während und nach dem Gebrauch verwendet werden können.

Die WO 2016/012368 A1 offenbart ein Verfahren zur Markierung von Substraten und markierten Behältern. Ein Verfahren umfasst die Schritte des Auftragens und Aushärtens einer Beschichtungszusammensetzung, die (a) ein matrixbildendes Material, das ein anorganisches Oxid oder einen anorganischen Oxidvorläufer umfasst, und (b) ein organisches Porenbildungsmittel umfasst. Die Markierung erfolgt durch Entfernen mindestens eines Teils des organischen Porenbildners an einer ausgewählten Stelle der Beschichtungsschicht. Die Markierung kann auf einem Behälter angebracht werden, in dem sich ein Produkt oder ein wärmeempfindliches Produkt befindet. Weitere Ausführungsformen beziehen sich auf mehrschichtige Beschichtungen und Verfahren zur Herstellung mehrschichtiger Beschichtungen.

Die WO 2009/156275 A1 offenbart Pigmentmischungen, die zwei unterschiedliche Komponenten A und B enthalten, wobei Komponente A ein Graphit in Form von Plättchen (Graphit-Nanoplättchen) ist, die eine mittlere Teilchengröße von unter 50 Mikron und eine Dicke von unter 100 nm aufweisen, und Komponente B ein organisches oder anorganisches Pigment ist. Die Verwendung von Graphit-Nanoplättchen in einer Pigmentmischung mit organischen und/oder anorganischen Pigmenten, insbesondere Effektpigmenten (Komponente B), ermöglicht die Herstellung metallisch anmutender Einfärbungen mit maximaler Deckkraft (Hintergrundsubstrat verschwindet vollständig) bei gleichzeitig gutem rheologischen Verhalten (Einsatz in geringer Konzentration).

Die WO 2009/124838 A2 beschreibt ein ophthalmologisches Implantat mit einer Markierung, die auch auf einem optisch abbildenden Element möglich ist. Allerdings wird zur Markierung in diesem Fall entweder ein fluoreszierender Farbstoff mit einem Emissionsmaximum außerhalb des für den Menschen sichtbaren Lichtspektrums oder ein absorbierender Farbstoff mit einem Absorptionsmaximum außerhalb des für den Menschen sichtbaren Lichtspektrums verwendet.

Die meisten Farbstoffe erfüllen allerdings nicht die Anforderungen an die Biokompatibilität, um für ophthalmologische Implantate verwendet werden zu können. Darüber hinaus erfordern Fluoreszenzfarbstoffe eine spezielle Ausrüstung, um gelesen zu werden, z. B. einen Anregungslaser mit der richtigen Wellenlänge. Außerdem können sie nur eine begrenzte Anzahl von Malen abgelesen werden, da die Moleküle nicht photostabil sind und bei Lichteinwirkung schnell ausbleichen. Damit ist beispielsweise die zuverlässige Identifizierung eines Implantats durch einen Arzt, der die Informationen erst Jahre nach der Operation abrufen muss, in der Regel nicht mehr möglich.

Neben dem Problem der unzureichenden Photostabilität sind organische Farbstoffe auch aufgrund ihrer chemischen Struktur in ihrer Anwendung stark eingeschränkt. Während UV- und UV-nahe Absorber relativ einfach zu realisieren sind, erfordern Strukturen, die bei längeren Wellenlängen, beispielsweise im für den Menschen sichtbaren Wellenlängenbereich zwischen etwa 380 nm und etwa 750 nm, absorbieren, entweder große konjugierte aromatische Verbindungen oder solche, die geladen sind. Erstere sind schwierig zu synthetisieren und bieten eine geringe Löslichkeit, während letztere bei Verwendung in einem Implantat das physiologische System stören können.

### Darstellung der Erfindung

Aufgabe der vorliegenden Erfindung ist es, eine verbesserte Möglichkeit zur Realisierung einer dauerhaften Produktkennzeichnung eines ophthalmologischen Implantats zu schaffen.

Die Aufgabe wird erfindungsgemäß durch ein ophthalmologisches Implantat gemäß Patentanspruch 1 gelöst. Vorteilhafte Ausgestaltungen mit zweckmäßigen Ausbildungen der Erfindung sind in den Unteransprüchen angegeben.

Ein erster Aspekt der Erfindung betrifft ein ophthalmologisches Implantat gemäß Anspruch 1 mit einem Grundkörper, welcher eine Produktkennzeichnung umfasst, wobei vorgesehen ist, dass die Produktkennzeichnung zumindest teilweise aus wenigstens einem Interferenzfarbstoff besteht. Mit anderen Worten ist es vorgesehen, dass das ophthalmologische Implantat auf seinem Grundkörper mit einer Produktkennzeichnung versehen ist, welche ihrerseits teilweise oder vollständig aus einem oder mehreren Interferenzfarbstoffen gebildet ist. Erfindungsgemäß ist es vorgesehen, dass der wenigstens eine Interferenzfarbstoff zumindest teilweise oder vollständig aus Nanocellulose, insbesondere Zellulosenanokristalle (CNC, NCC), Zellulosenanofasern (CNF), nanofibrillierter Zellulose (NFC) und/oder bakterieller Nanocellulose, besteht. Besonders bevorzugt sind dabei Zellulosenanokristalle und/oder Zellulosenanofasern. Nanocellulose ist ein Material mit besonders vorteilhaften optischen, mechanischen und chemischen Eigenschaften. Cellulose-Nanokristalle (CNCs) wurden als verantwortlich für die Erzeugung der leuchtenden, schillernden Farben von verschiedenen Pflanzen entdeckt. Dabei sind die Farben wie bereits beschrieben nicht auf das Vorhandensein eines bestimmten Pigments zurückzuführen, sondern auf die Wechselwirkung zwischen dem Licht und den periodischen Mikrostrukturen der Nanocellulose in Blütenblättern und Blättern, weshalb Interferenzfarben auch "Strukturfarben" genannt werden. Zellulose ist an sich zudem ein biokompatibles Material biologischen Ursprungs, das vom menschlichen Körper abgebaut und daher problemlos und normalerweise ohne weitere Zusatzbehandlungen wie Immobilisierung oder Einkapselung zur teilweisen oder vollständigen Realisierung der Produktkennzeichnung verwendet werden kann. Zellulose ist weithin kostengünstig und aus nachhaltigen Quellen verfügbar. Mit der Verwendung von Nanocellulose entfällt im Vergleich zu Absorptions- und Fluoreszenzfarbstoffen zudem die Notwendigkeit von speziellen Lese- oder Auswertegeräten.

Unter einem Interferenzfarbstoff wird im Rahmen der vorliegenden Offenbarung also eine Verbindung verstanden, welche aufgrund ihrer Struktur bei gewissen Wellenlängen, die im einfallenden Lichte vertreten sind, durch Interferenz wechselwirkt. Das physikalische Prinzip hinter Strukturfarben ist also nicht die Absorption, sondern die Beugung. Dieses Phänomen tritt auf, wenn Licht bzw. elektromagnetische Strahlung mit periodischen Strukturelementen des Interferenzfarbstoffs in Wechselwirkung tritt, wobei der Abstand (Periodizität) zwischen den Strukturelementen des Interferenzfarbstoffs in der gleichen Größenordnung wie die einfallende(n) Wellenlänge(n) liegt. Die spezifische(n) Wellenlänge(n), die diese Abstandsbedingung(en) erfüllt bzw. erfüllen, wird bzw. werden von den periodischen Strukturelementen gestreut, wodurch die Wellen konstruktiv und/oder destruktiv überlagern. Das resultierende Licht wird vom Interferenzfarbstoff reflektiert und kann als Farbeindruck wahrgenommen werden. Die Bedingung der konstruktiven Interferenz hängt dabei vom Abstand der Strukturelemente (d), der Wellenlänge des einfallenden Lichts (λ) und dem Einfallswinkel (θ) ab, für den die konstruktive Interferenz am stärksten ist. Dieser Zusammenhang kann mit der Formel nλ = 2d sin θ (Bragg'sche Beugung) beschrieben werden, in welcher n eine natürliche Zahl ist, die die Beugungsordnung angibt. Der Ursprung der Farbe liegt in diesem Fall also nicht in einem spezifischen elektronischen Übergang in den Molekülen wie beispielsweise bei Fluoreszenzfarbstoffen, sondern in einer bestimmten räumlichen Organisation der Strukturelemente des Interferenzfarbstoffs, die damit als eine Art Gitter wirken. Ein Interferenzfarbstoff bietet damit erhebliche Vorteile gegenüber herkömmlichen Absorptions- bzw. Fluoreszenzfarbstoffen, da diese aufgrund der Empfindlichkeit des Moleküls im angeregten Zustand gegenüber oxidativem Stress einem chemischen Abbau unterliegen. Dagegen bietet die Verwendung eines Interferenzfarbstoffs eine deutlich höhere Gestaltungsfreiheit und eine gleichbleibende Komplexität, die nahezu unabhängig von der gewählten Detektions- bzw. Zielwellenlänge ist. Zudem sind Interferenzfarbstoffe wesentlich photostabiler als Absorptions- bzw. Fluoreszenzfarbstoffe und können sehr präzise an gewünschte Wellenlängen bzw. Wellenlängenbereiche angepasst werden. Durch die Verwendung des wenigstens einen nicht-ausbleichenden Strukturfarbstoffs zur teilweisen oder vollständigen Realisierung der Produktkennzeichnung, kann diese beispielsweise mit einer einfachen weißen Lichtquelle, das heißt mit sehr geringem apparativen Aufwand, oder alternativ bei bestimmten Wellenlängen ausgelesen werden. Durch die einfache Anpassbarkeit von Strukturfarbstoffen kann die Produktkennzeichnung zudem besonders einfach an eine gewünschte Wellenlänge bzw. einen gewünschten Wellenlängenbereich angepasst werden. Schließlich kann auch die Position der Produktkennzeichnung auf dem Implantat besonders flexibel gewählt werden. Vorzugsweise wird die Produktkennzeichnung ausschließlich aus einem oder mehreren Interferenzfarbstoffen, die auch als strukturelle Farben bezeichnet werden, gebildet. Alternativ kann vorgesehen sein, dass die Produktkennzeichnung eine oder mehrere weitere Verbindungen, die keine strukturelle Farbe ist bzw. sind, umfasst. In einer weiteren Ausgestaltung kann die Produktkennzeichnung zusätzlich zu einem oder mehreren Interferenzfarbstoffen auch andere Strukturelemente wie etwa relative Vertiefungen und/oder relative Erhöhungen bezüglich einer Oberfläche des Grundkörpers umfassen, durch welche Informationen codiert sind.

Die Produktkennzeichnung des erfindungsgemäßen ophthalmologischen Implantats ist dabei vorzugsweise maschinenlesbar und damit auch maschinenauswertbar ausgebildet. Hierdurch kann ein maschineller Ermittlungs- und Prüfprozess durchgeführt werden, wodurch eine zuverlässigere und komfortablere Handhabung des erfindungsgemäßen Implantats ermöglicht ist. Beispielsweise kann die erfindungsgemäße Produktkennzeichnung mit einem einfachen Operationsmikroskop ausgelesen werden, ohne dass wie bei Fluoreszenzfarbstoffen ein Laser mit speziellen Wellenlängen verwendet werden muss. In weiterer Ausgestaltung kann ein geeigneter, an sich bekannter Polarisator im optischen Ausleseweg angeordnet werden.

Als Strukturfarbstoffe können grundsätzlich alle geeigneten Verbindungen, die gegebenenfalls als Nanopartikel vorliegen, verwendet werden. Generell eignen sich viele Nanopartikel, da sie vergleichsweise einfach zu synthetisieren sind und aufgrund ihrer geometrischen Eigenschaften eine einfache Abstimmung auf bestimmte Wellenlängen ermöglichen. Bei der Verwendung von nicht oder eingeschränkt biokompatiblen Nanopartikeln, beispielsweise Metall- oder Halbleiterpartikeln, kann die Biokompatibilität beispielsweise durch Immobilisierung und/oder Einkapselung verbessert werden.

In einer vorteilhaften Ausgestaltung der Erfindung ist vorgesehen, dass der wenigstens eine Interferenzfarbstoff Partikel und/oder Partikelkonglomerate enthält, die eine mittlere Größe von mindestens 40 µm, insbesondere von mindestens 50 µm besitzen, um einen Farbeindruck im für den Menschen sichtbaren Wellenlängenbereich zwischen etwa 380 nm und etwa 750 nm zu erzeugen. Mit anderen Worten ist es vorgesehen, dass Interferenzfarbstoff Partikel aufweist, deren Größe im angegebenen Nanometerbereich liegt und/oder die in entsprechend großen Konglomeraten, das heißt in größeren Ansammlungen oder "Baugruppen" angeordnet sind. Ein Partikelkonglomerat ("Baugruppe") enthält also mehrere, in einer bestimmten Struktur zusammengelagerte Partikel, beispielsweise Zellulose-Nanokristallgruppen. Die Partikel und/oder Partikelkonglomerate haben eine Mindestgröße von 40 µm, können aber auch größer sein. Diese Mindestgröße der Partikel bzw. Partikelkonglomerate gewährleistet eine ausreichende Periodizität der Struktur, um mit einstrahlendem Licht konstruktiv interferieren zu können.

Alternativ oder zusätzlich ist es vorgesehen, dass der Interferenzfarbstoff selbstorganisationsfähige Partikel und/oder Partikelkonglomerate umfasst, die dazu ausgebildet sind, sich in einer geordneten, insbesondere periodischen Struktur anzuordnen. Hierdurch erfolgt automatisch eine für den gewünschten Farbeindruck benötigte Ausrichtung und Anordnung der einzelnen Partikel bzw. Strukturelemente des Interferenzfarbstoffs beim Auftragen auf den Grundkörper. Im Rahmen der vorliegenden Offenbarung werden mit dem Begriff "Partikel" generell auch Fasern bezeichnet, bei welchen es sich um Partikel mit einer länglichen Form handelt. Weiterhin kann der Begriff "Partikel" im Rahmen der vorliegenden Offenbarung auch unrunde, ovale und unregelmäßig geformte Strukturelemente bezeichnen.

Weitere Vorteile ergeben sich dadurch, dass der wenigstens eine Interferenzfarbstoff auf dem Grundkörper und/oder innerhalb des Grundkörpers und/oder auf einer Beschichtung des Grundkörpers und/oder innerhalb einer Beschichtung des Grundkörpers und/oder unter einer Beschichtung des Grundkörpers angeordnet ist. Hierdurch kann der Interferenzfarbstoff besonders flexibel angeordnet werden. Insbesondere wenn der wenigstens eine Interferenzfarbstoff innerhalb des Grundkörpers, innerhalb einer Beschichtung des Grundkörpers und/oder unter einer Beschichtung des Grundkörpers angeordnet ist, ist dabei ein besonders hoher Schutz vor mechanischer Beschädigung gegeben, wodurch die Produktkennzeichnung besonders dauerhaft ausgebildet und entsprechend lange nach der ursprünglichen Operation noch zuverlässig ausgelesen werden kann. Die Beschichtung kann vorzugsweise als Schutzschicht ausgebildet sein und/oder besonders bevorzugt einen Brechungsindex aufweisen, der zumindest im Wesentlichen dem des Kammerwassers entspricht. Hierzu eignen sich beispielsweise fluorierte oder perfluorierte (Meth)Acrylate. Auf diese Weise können unerwünschte optische Brechungseffekte an den Phasengrenzen vermieden werden. Bedarfsweise kann der Interferenzfarbstoff auch kovalent an das Material des Grundkörpers und/oder der Beschichtung gebunden sein.

In einer weiteren vorteilhaften Ausgestaltung der Erfindung ist vorgesehen, dass die Produktkennzeichnung einen Implantattyp und/oder eine optische Eigenschaft, insbesondere eine Brechkraft, des ophthalmologischen Implantats und/oder einen Datenbankschlüssel und/oder eine Positionierungsinformation charakterisiert. Die Produktkennzeichnung enthält mit anderen Worten Informationen, die Spezifikationsdaten des individuellen ophthalmologischen Implantats, beispielsweise Modellnummer, Dioptrie, Typ, Hersteller, Modell, Version, Material, torische Achse usw. charakterisiert. Die Spezifikationsdaten können aber auch durch den Datenbankschlüssel als eindeutige Kennung (UID bzw. UUID) repräsentiert werden, die den Abruf entsprechender Produktinformationen aus einer Datenbank ermöglicht. Die Produktkennzeichnung kann darüber hinaus geometrische Daten des einzelnen Implantats charakterisieren, die eine maschinelle Erkennung seiner Position ermöglichen. Aufgrund der kodierten Natur der Produktkennzeichnung kann dabei selbst eine Untergruppe von erkannten Merkmalen nützliche Positionierungsinformation wiedergeben, beispielsweise um das Implantat präziser im Auge auszurichten zu können. Die Produktkennzeichnung kann dabei auch Informationen zur Fehlererkennung, Fehlertoleranz und/oder Fehlerkorrektur charakterisieren.

In einer weiteren vorteilhaften Ausgestaltung der Erfindung ist vorgesehen, dass die Produktkennzeichnung wenigstens einen runden und/oder quadratischen und/oder länglichen und/der geometrisch unregelmäßigen Markierungspunkt aus dem wenigstens einen Interferenzfarbstoff umfasst und/oder dass die Produktkennzeichnung mehrere Markierungspunkte in Form eines linearen Barcodes und/oder eines 2D-Matrixcodes und/oder eines 3D-Matrixcodes und/oder eines Punktrasters mit einem pseudozufälligen unregelmäßigen Charakter umfasst. Unter einem Markierungspunkt ist dabei kein Punkt im mathematischen Sinn gemeint, sondern ein Markierungspunkt, der eine bestimmte Fläche besitzt. In einfachster Ausgestaltung ist ein Markierungspunkt rund und besitzt einen bestimmten (mittleren) Durchmesser. Alternativ kann ein Markierungspunkt aber auch eine unrunde Form besitzen, beispielsweise eine elliptische, quadratische, längliche und/oder geometrisch unregelmäßige Form. Im Allgemeinen kann die Produktkennzeichnung jede Art von visuell erkennbarer kodierter Information sein. Beispiele für solche kodierten Informationen sind lineare Barcodes, 2D-Matrix-Barcodes einschließlich Dotcodes oder QR-Codes oder erweiterte Codes wie 3D-Matrixcodes. Codes können in der Form, Anzahl, Größe oder Breite der (einzelnen) Markierungspunkt, der gesamten Codegröße, dem Abstand zwischen den Markierungspunkten und der Ausrichtung der Markierungspunkte innerhalb des Codes variieren. "Pseudozufällig unregelmäßig" bedeutet im Rahmen der vorliegenden Offenbarung, dass eine Anzahl verschiedener definierter Abweichungen der Markierungspunkte von einem festen Bezugspunkt, der einen regelmäßigen Charakter Musters erzeugen würde, besteht. Dadurch können Gitterbeugungseffekte minimieren werden, die zu einer Beeinträchtigung des Sehens führen könnten. Die Markierungspunkte der Produktkennzeichnung sind durch ihre Farbe, Struktur und/oder Anordnung vorzugsweise derart ausgelegt, dass sie keine für den Patienten wahrnehmbare negative optische Wirkung haben.

Weitere Vorteile ergeben sich dadurch, dass der wenigstens eine Interferenzfarbstoff mit Licht im Wellenlängenbereich zwischen 300 nm und 3000 nm, insbesondere im Wellenlängenbereich zwischen 400 nm und 750 nm und/oder im Wellenlängenbereich zwischen etwa 750 nm und etwa 3000 nm, wechselwirkt. Eine besonders einfache Ermittlung und Auswertung der Produktkennzeichnung ist dabei möglich, wenn der wenigstens eine Interferenzfarbstoff durch Wechselwirkung mit Licht im Wellenlängenbereich zwischen 300 nm und 900 nm, insbesondere im für den Menschen sichtbaren Wellenlängenbereich zwischen etwa 400 nm und 750 nm, einen Farbeindruck erzeugt. Grundsätzlich können aber auch Interferenzfarbstoffe mit einem hohen Reflexionsgrad im nahen Infrarotbereich zwischen etwa 750 nm bis etwa 3000 nm sehr vorteilhaft sein, insbesondere da außerhalb des für den Menschen wahrnehmbaren Wellenlängenbereichs keine Gefahr einer optischen Beeinträchtigung für den Patienten besteht.

In einer weiteren vorteilhaften Ausgestaltung der Erfindung ist vorgesehen, dass die Produktkennzeichnung mindestens zwei Interferenzfarbstoffe mit unterschiedlichen Farben umfasst. Unter Interferenzfarbstoffen mit unterschiedlichen Farben sind dabei Interferenzfarbstoffe zu verstehen, die aufgrund ihres unterschiedlichen strukturellen Aufbaus mit unterschiedlichen Wellenlängen des eingestrahlten Lichts wechselwirken und damit unter ansonsten gleichen oder vergleichbaren Beleuchtungsbedingungen zu unterschiedlichen Farbeindrücken führen. Dies stellt eine besonders vorteilhafte Möglichkeit zur hochdimensionalen Kodierung von Informationen dar, da nicht nur die relativen Positionen der unterschiedlichen Interferenzfarbstoffe bezüglich des Grundkörpers, sondern auch deren jeweilige Farben ermittelt und ausgewertet werden können.

In einer weiteren vorteilhaften Ausgestaltung der Erfindung ist vorgesehen, dass das ophthalmologische Implantat als Kapselspannring oder als Stent oder als Intraokularlinse oder als implantierbare Kontaktlinse (ICL) ausgebildet ist. Hierdurch können die erfindungsgemäßen Vorteile für unterschiedliche Implantattypen realisiert werden.

Weitere Vorteile ergeben sich dadurch, dass der Grundkörper wenigstens einen optischen und wenigstens einen haptischen Teil umfasst, wobei die Produktkennzeichnung auf dem wenigstens einen optischen Teil und/oder auf dem wenigstens einen haptischen Teil angeordnet ist. Hierdurch ist eine besonders hohe Freiheit bei der Anordnung der Produktkennzeichnung gegeben. Die Produktkennzeichnung kann grundsätzlich auch aus mehreren Teilen bestehen, die an jeweils unterschiedlichen Stellen des Grundkörpers angeordnet sind. Ebenso kann die Produktkennzeichnung teilweise oder vollständig in einem Übergangsbereich zwischen dem optischen und dem haptischen Teil angeordnet sein. Vorzugsweise ist die Produktkennzeichnung nicht in einer zentralen optischen Zone des optischen Teils angeordnet. Die zentrale optische Zone ist für Intraokularlinsen definiert als der zentrale Bereich mit einem Durchmesser von 4,4 mm. In diesem Bereich ist eine Markierung nach dem derzeitigen Stand nicht ISO-konform. Die Produktkennzeichnung kann aber vorzugsweise in der peripheren optischen Zone, das heißt im äußeren Bereich des optischen Teils eines ophthalmologischen Implantats angeordnet sein, wodurch die zentrale optische Zone mit einem Durchmesser von 4,4 mm frei bleibt. In diesem peripheren Bereich ist eine Markierung nach dem derzeitigen Stand ISO-konform.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische Ansicht eines ophthalmologischen Implantats gemäß einer ersten Ausführungsform;
- Fig. 2: eine schematische Ansicht des ophthalmologischen Implantats gemäß einer zweiten Ausführungsform;
- Fig. 3: eine schematische Ansicht des ophthalmologischen Implantats gemäß einer dritten Ausführungsform;
- Fig. 4: eine Veranschaulichung des Funktionsprinzips und einer Auslesemethode, die auf einer spektralen Charakterisierung einer erfindungsgemäßen Produktkennzeichnung beruhen;
- Fig. 5: eine weitere Ausführungsform einer farb- und ortscodierten Produktkennzeichnung des erfindungsgemäßen ophthalmologischen Implantats;
- Fig. 6: eine weitere Ausführungsform der farb- und ortscodierten Produktkennzeichnung des erfindungsgemäßen ophthalmologischen Implantats;
- Fig. 7: eine weitere Ausführungsform der Produktkennzeichnung des erfindungsgemäßen ophthalmologischen Implantats mit torischen Markern als Ausrichtungshilfe;
- Fig. 8: eine weitere Ausführungsform der Produktkennzeichnung des erfindungsgemäßen ophthalmologischen Implantats;
- Fig. 9: eine weitere Ausführungsform der Produktkennzeichnung des erfindungsgemäßen ophthalmologischen Implantats; und
- Fig. 10: eine weitere Ausführungsform des erfindungsgemäßen ophthalmologischen Implantats mit einem optischen und zwei haptischen Teilen.

### Bevorzugte Ausführung der Erfindung

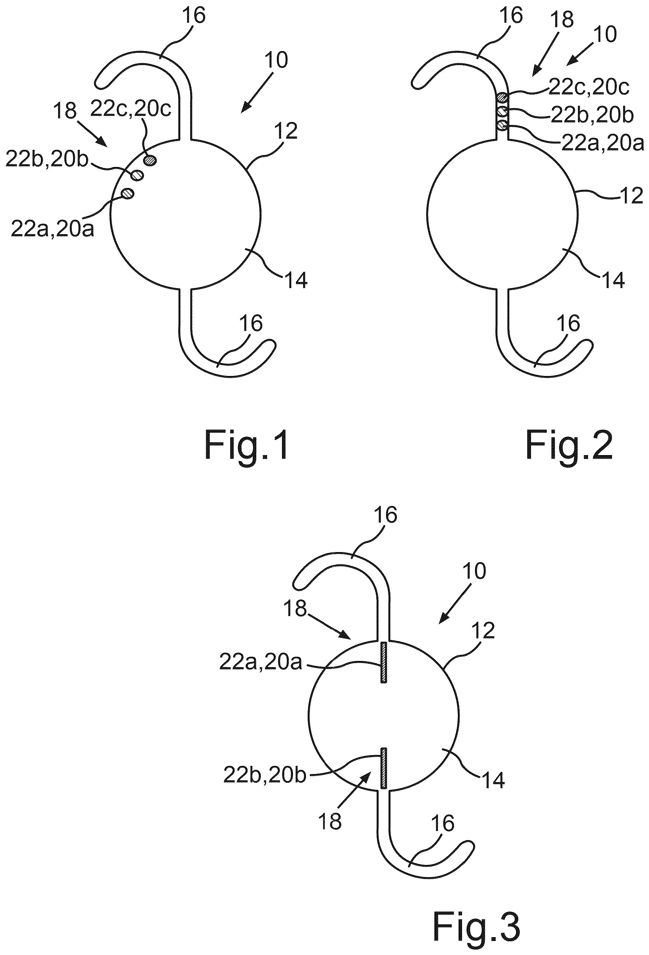

Fig. 1 zeigt eine schematische Ansicht eines ophthalmologischen Implantats 10 gemäß einer ersten Ausführungsform. Das ophthalmologische Implantat 10, welches vorliegend exemplarisch als Intraokularlinse ausgebildet ist, besitzt einen Grundkörper 12, welcher im gezeigten Beispiel einen optischen Teil 14 und zwei haptische Teile 16 umfasst. Es ist zu betonen, dass die vorliegende Erfindung nicht auf Intraokularlinsen beschränkt ist. Ebenso ist es nicht erforderlich, dass ein erfindungsgemäßes Implantat 10 einen optischen Teil 14 und zwei haptische Teile 16 aufweist. Beispielsweise kann der Grundkörper 12 auch nur einen haptischen Teil 16 aufweisen, der gegebenenfalls den optischen Teil 14 umgibt. Ebenso ist es möglich, dass das erfindungsgemäße Implantat 10 bzw. sein Grundkörper 12 nur einen oder mehrere haptische Teile 16 oder nur einen oder mehrere optische Teile 14 besitzt.

Im gezeigten Beispiel weist der Grundkörper 12 ISO-konform in einem peripheren Bereich des optischen Teils 14 eine maschinenlesbare Produktkennzeichnung 18 auf, die exemplarisch aus drei Markierungspunkten 20a, 20b, 20c mit jeweils unterschiedlicher Position auf dem Grundkörper 12 besteht. Die Produktkennzeichnung 18 charakterisiert bzw. kodiert wichtige Informationen über das Implantat 10, die für Ärzte und Patienten vor, während und nach der Operation zugänglich sind. Die Markierungspunkte 20a, 20b, 20c sind deshalb ihrerseits aus drei unterschiedlichen Interferenzfarbstoffen 22a, 22b, 22c mit jeweils unterschiedlicher Farbe gebildet. Die unterschiedlichen Farben der Interferenzfarbstoffe 22a, 22b, 22c sind in den Figuren schematisch mit unterschiedlichen Füllmustern angedeutet. Jeder Markierungspunkt 20a, 20b, 20c kann für ein oder mehrere Merkmale des Implantats 10 stehen, beispielsweise für eine Modellbezeichnung und/oder für die sphärischen und/oder torische Dioptrien, die anhand der Farbe und/oder der Position auf dem Grundkörper 12 des jeweiligen Markierungspunkt 20a, 20b, 20c klassifiziert bzw. kodiert werden.

Als Interferenzfarbstoffe 22a, 22b, 22c werden drei Nanocellulosen mit jeweils unterschiedlichen Mikrostrukturen verwendet. Nanocellulose besitzt unter den Nanomaterialien besonders vorteilhafte optische, mechanische, chemische und biologische Eigenschaften. Als Nanocellulose können insbesondere Cellulose-Nanokristalle (CNCs) verwendet werden. Die resultierenden Farbeindrücke sind damit nicht auf das Vorhandensein eines bestimmten Pigments zurückzuführen, sondern auf die Wechselwirkung zwischen dem eingestrahlten Licht und den jeweiligen periodischen Mikrostrukturen der drei Nanocellulosen, die jeweils als eine Art Beugungsgitter fungieren und dementsprechend mit unterschiedlichen Wellenlängen bzw. Wellenlängenbereichen des eingestrahlten Lichts wechselwirken und unterschiedliche Farbeindrücke erzeugen.

Die Interferenzfarbstoffe 22a, 22b, 22c stellen als Strukturfarben nicht-ausbleichende Farbstoffe dar, die sich dementsprechend hervorragend für die Herstellung einer Produktkennzeichnung 18 des erfindungsgemäßen ophthalmologischen Implantats 10 eignen, da sie je nach Mikrostruktur ohne großen apparativen Aufwand mit einer einfachen weißen Lichtquelle oder alternativ bei bestimmten Wellenlängen, beispielsweise mit Nahinfrarot-Strahlung im Wellenlängenbereich zwischen 750 nm und 3000 nm, ausgelesen und entsprechend einfach ausgewertet werden können. Die Interferenzfarbstoffe 22a, 22b, 22c und insbesondere Nanocellulosen können dabei frei auf die gewünschten Wellenlängen abgestimmt werden. Entsprechend können auch die Art und Position der Produktkennzeichnung 18 auf dem erfindungsgemäßen Implantat 10 nach Bedarf frei gewählt werden.

Die verwendeten Nanocellulosen bestehen aus Partikelkonglomeraten bzw. Faserkonglomeraten, die eine mittlere Größe von mindestens 50 µm besitzen und periodisch organisiert sind, so dass konstruktive Interferenz auftreten kann. Mindestens 40 µm, vorzugsweise mindestens 50 µm stellt eine Größe der Nanokristalle dar, die ausreichend ist, um genügend Licht im für den Menschen sichtbaren Wellenlängenbereich zu reflektieren. Daher können die Nanocellulosepartikelkonglomerate eine Größe von 40-50 µm haben, aber aus mehreren Nanokristallen bzw. Partikeln mit geringerer Größe bestehen. Darüber hinaus besitzen die verwendeten Nanocellulose-Partikel bzw. -Fasern eine Selbstorganisationsfähigkeit, wodurch sich beim Aufbringen auf den Grundkörper 12 selbständig in einer periodischen und damit zumindest überwiegend geordneten und nicht-amorphen Struktur anordnen.

Die Produktkennzeichnung 18 besitzt damit verschiedene Vorteile. Einerseits sorgen die Interferenzfarbstoffe 22a, 22b, 22c für eine dauerhafte Färbung ohne kein Ausbleichen, andererseits können beispielsweise gegenüber einem QR-Code, einem Punktgitter oder dergleichen durch Ausnutzung der Farbe als Informationsträger mehr Informationen auf weniger Raum kodiert werden. Außerdem müssen die Markierungspunkte 20a, 20b, 20c nicht besonders große geformt sein, sondern nur groß genug, um die innere Struktur der Interferenzfarbstoffe 22a, 22b, 22c, die die gewünschte Farbe hervorbringt, zu erhalten (Partikelgröße ca. 50 µm oder mehr). Die Form der Markierungspunkte 20a, 20b, 20c kann davon abgesehen rund, oval, quadratisch, länglich oder geometrisch zufällig sein.

Eine große Verbesserung, die das erfindungsgemäße Implantat 10 bietet, betrifft die Einfachheit des Ausleseverfahrens der kodierten Informationen, da die Färbung der Produktkennzeichnung 18 nicht auf der Anregung eines Fluoreszenzfarbstoffs mit einer bestimmten Wellenlänge beruht, sondern allein auf der Fähigkeit der Interferenzfarbstoffe 22a, 22b, 22c, ausgewählte Wellenlängen des sichtbaren Spektrums gebeugt zu reflektieren. Das einfallende Licht für die Untersuchung der Produktkennzeichnung 18 kann daher eine einfache weiße Lichtquelle sein, wie z. B. eine Halogenlampe. Das reflektierte Licht kann ebenso einfach beispielsweise mit einer mit einem Polarisator ausgestatteten Kamera ausgelesen werden. Da keine spezielle Ausrüstung erforderlich ist, können Ärzte die Ablesung der Produktkennzeichnung 18 bei Routineuntersuchungen durchführen.

Fig. 2 zeigt eine schematische Ansicht des ophthalmologischen Implantats 10 gemäß einer zweiten Ausführungsform. Im Unterschied zum vorherigen Ausführungsbeispiel ist die Produktkennzeichnung 18 ausschließlich auf einem der zwei haptischen Teile 16 des Grundkörpers 12 angeordnet. Alternativ könnte die Produktkennzeichnung 18 natürlich auch auf beiden haptischen Teilen 16 und/oder optional auch auf dem optischen Teil 14 vorgesehen sein.

Fig. 3 zeigt eine schematische Ansicht des ophthalmologischen Implantats 10 gemäß einer dritten Ausführungsform. Die Produktkennzeichnung 18 ist dabei als torischer Marker, das heißt als Positionierungsinformation ausgebildet und befindet sich an gegenüberliegenden Rändern des optischen Teils 14, jeweils nahe der beiden haptischen Teile 16 des ophthalmologischen Implantats 10. Die torischen Marker 18 bestehen aus länglichen Markierungspunkten 20a, 20b, die ihrerseits erneut aus Interferenzfarbstoffen 22a, 22b gebildet werden. Die Interferenzfarbstoffe 22a, 22b können dabei grundsätzlich gleich bzw. gleichfarbig oder unterschiedlich bzw. verschiedenfarbig ausgebildet sein.

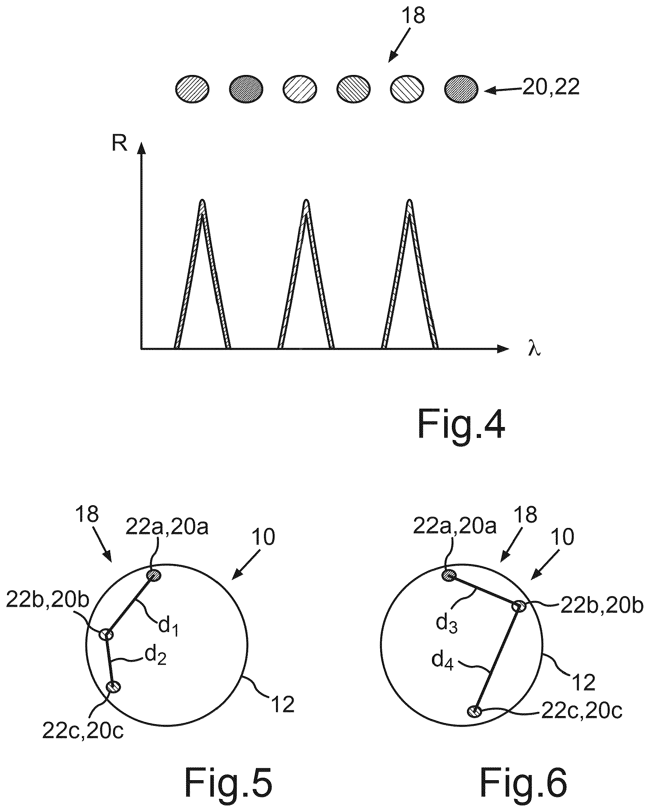

Fig. 4 das Funktionsprinzip und eine Auslesemethode, die auf der spektralen Charakterisierung der Produktkennzeichnung 18 beruht. Auf der Abszisse des Diagramms von Fig. 4 ist die Wellenlänge λ und auf der Ordinate die Intensität der reflektierten Strahlung R (rel. Einheit) aufgetragen ist. Farben, die das für den Menschen sichtbare Spektrum abdecken, können mit unterschiedlichen bzw. unterschiedlich organisierten Nanocellulosekristallen erzeugt und anhand der Position des Maximums der Reflexion R auf der Wellenlängenachse λ ermittelt werden. Das Maximum des Reflexionsvermögens wird vorzugsweise bei einem konstanten Streuwinkel θ bzw. dem Streuwinkel θ der höchsten Intensität ausgelesen. Es gibt auch alternative Auslesemethoden, die auf RGB-Kameras und der Zerlegung der Intensität in diese drei Kanäle beruhen. Auch andere Methoden sind denkbar. Damit können unterschiedliche Markierungspunkte 20 realisiert werden, die jeweils aus einem Interferenzfarbstoff 22 bestehen, wobei die Markierungspunkte 20 bzw. die Interferenzfarbstoffe 22 unterschiedliche Farbeindrücke erzeugen, die in Fig. 4 mit unterschiedlichen Schraffuren angedeutet sind.

Fig. 5 zeigt eine weitere Ausführungsform einer farb- und ortscodierten Produktkennzeichnung 18 des erfindungsgemäßen ophthalmologischen Implantats 10. Die Produktkennzeichnung 18 besteht exemplarisch aus drei Markierungspunkten 20a-20c, die ihrerseits jeweils aus einem Interferenzfarbstoff 22a-22c bestehen. Man erkennt, dass die Markierungspunkte 20a-20b durch den Vektor d₁ und die Markierungspunkte 20b-20c durch den Vektor d₂ verbunden sind.

Fig. 6 zeigt eine weitere Ausführungsform der farb- und ortscodierten Produktkennzeichnung 18 des erfindungsgemäßen ophthalmologischen Implantats 10. Auch hier besteht die Produktkennzeichnung 18 exemplarisch aus drei Markierungspunkten 20a-20c, die ihrerseits jeweils aus einem Interferenzfarbstoff 22a-22c bestehen. Man erkennt, dass die Markierungspunkte 20a-20b durch den Vektor d₃ und die Markierungspunkte 20b-20c durch den Vektor d₄ verbunden sind. Die Vektoren d₃ und d₄ unterscheiden sich von den Vektoren d₁ und d₂ aus Fig. 5, so dass über die Position der Markierungspunkten 20a-20c auf dem Implantat, die Farbe der Interferenzfarbstoffe 22a-22c und die Vektoren d₁-d₄ bzw. den Abstand zwischen den einzelnen Markierungspunkten 20a-20c, das heißt über die relative Position der Markierungspunkten 20a-20c zueinander, eine kompakte Kodierung von Informationen ermöglicht ist.

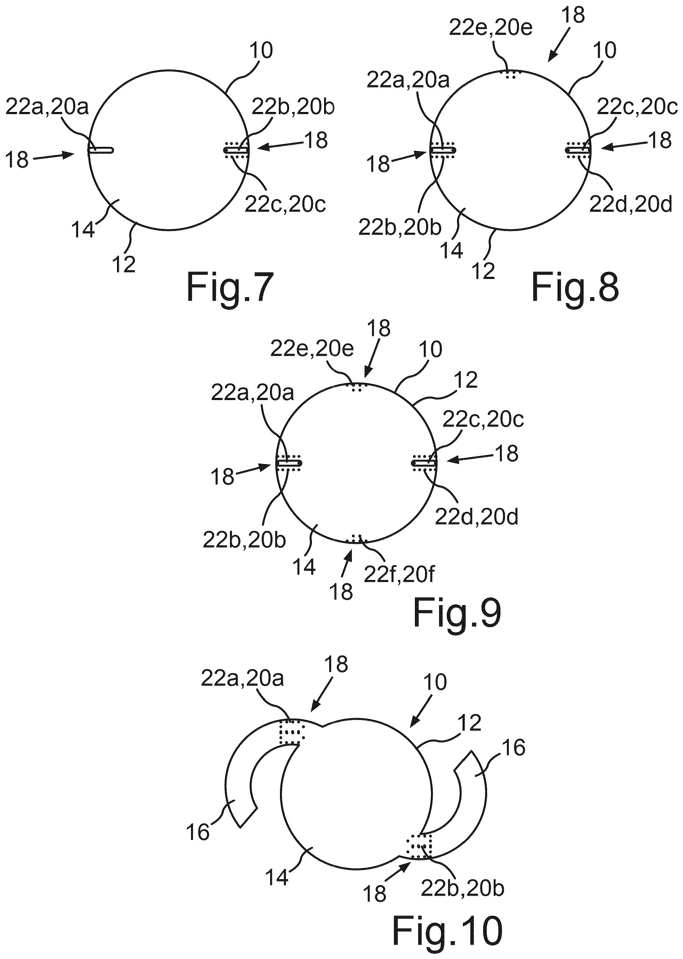

Fig. 7 zeigt eine weitere Ausführungsform der Produktkennzeichnung 18 des erfindungsgemäßen ophthalmologischen Implantats 10 mit torischen Markern 20a, 20b aus Interferenzfarbstoffen 22a, 22b als Ausrichtungshilfe. Die Produktkennzeichnung 18 befindet sich im peripheren Bereich des optischen Teils 14. Ein oder mehrere haptische Teile 16 können ebenfalls vorgesehen sein. Über und unter dem torischen Marker 20b sind weitere Markierungspunkte 20c aus einem oder mehreren Interferenzfarbstoffen 22c angeordnet, um zusätzliche Informationen über die ophthalmologische Linse 10 zu kodieren.

Fig. 8 zeigt eine weitere Ausführungsform der Produktkennzeichnung 18 des erfindungsgemäßen ophthalmologischen Implantats 10. Im Unterschied zum vorhergehenden Ausführungsbeispiel weist die Produktkennzeichnung 18 oberhalb und unterhalb der beiden torischen Marker 20a, 20c weitere Markierungspunkte 20b, 20d aus einem oder mehreren Interferenzfarbstoffen 22b, 22d auf, um zusätzliche Informationen über die ophthalmologische Linse 10 zu kodieren. Zusätzlich zu den Markierungspunkten 20a, 2b und 20c, 20d, die sich im vorliegenden Beispiel formal in der 9- bzw. 3-Uhr-Position des Implantats 10 befinden, ist ein zusätzlicher Markierungspunkt 20e aus einem Interferenzfarbstoff 22e in der 12-Uhr-Position des ophthalmologischen Implantats 10 vorgesehen. Hierdurch kann eine zusätzlich verbesserte Ausrichtungshilfe realisiert werden.

Fig. 9 zeigt eine weitere Ausführungsform der Produktkennzeichnung 18 des erfindungsgemäßen ophthalmologischen Implantats 10, die im Wesentlichen der vorhergehenden Ausführungsform entspricht. Gegenüber der in Fig. 8 gezeigten Ausführungsform weist die Produktkennzeichnung 18 einen zusätzlichen Markierungspunkt 20f aus einem Interferenzfarbstoff 22f in der 6-Uhr-Position des optischen Teils 14 des ophthalmologischen Implantats 10 auf.

Fig. 10 zeigt eine weitere Ausführungsform des erfindungsgemäßen ophthalmologischen Implantats 10 mit einem optischen Teil 14 und zwei haptischen Teilen 16. Dabei befindet sich die Produktkennzeichnung 18 jeweils im Anbindungsbereich der haptischen Teile 16 an den optischen Teil 14, aber außerhalb einer optischen Zone des optischen Teils 14.

Die Produktkennzeichnung 18 ermöglicht damit nicht nur eine zuverlässige Identifizierung des ophthalmologischen Implantats 10, sondern auch die Erkennung der Position bzw. räumlichen Ausrichtung des Implantats 10 während der Operation. Die Verwendung der Produktkennzeichnung 18 ermöglicht die Bereitstellung von Spezifikationsdaten und tatsächlichen Positionsdaten. Die von der Produktkennzeichnung 18 gelieferten Informationen eröffnen neue Möglichkeiten für die computergestützte Optimierung der Positionierung des Implantats 10. Aufgrund des codierten Charakters der Produktkennzeichnung 18 liefert selbst eine Untergruppe der erkannten Merkmale hilfreiche Informationen, um das Implantat 10 stabiler und präziser im Auge auszurichten. Die Produktkennzeichnung 18 umfasst daher vorzugsweise Mittel zur Fehlererkennung, Fehlertoleranz und idealerweise zur Fehlerkorrektur. Durch die Verwendung des oder der Interferenzfarbstoffe 22 ist zudem eine einfache, zuverlässige und dauerhafte Ermittlung der kodierten Informationen sichergestellt.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, DIN-Toleranzen und dergleichen - als vom Rahmen der Erfindung mitumfasst anzusehen.

### Bezugszeichenliste

- 10: Implantat
- 12: Grundkörper
- 14: optischer Teil
- 16: haptischer Teil
- 18: Produktkennzeichnung
- 20: Markierungspunkt
- 22: Interferenzfarbstoff
- d₁, d₂, d₃, d₄: Vektor
- λ: Wellenlänge
- R: Reflexion

## Patentansprüche

1. Ophthalmologisches Implantat (10) mit einem Grundkörper (12), welcher eine vorzugsweise maschinenlesbare Produktkennzeichnung (18) umfasst, wobei die Produktkennzeichnung (18) zumindest teilweise aus wenigstens einem Interferenzfarbstoff (22) besteht
**dadurch gekennzeichnet, dass**
der wenigstens eine Interferenzfarbstoff (22) zumindest teilweise aus Nanocellulose, insbesondere Zellulosenanokristalle, Zellulosenanofasern, nanofibrillierter Zellulose und/oder bakterieller Nanocellulose, besteht.

2. Ophthalmologisches Implantat (10) nach Anspruch 1,
**dadurch gekennzeichnet, dass**
der wenigstens eine Interferenzfarbstoff (22) Partikel und/oder Partikelkonglomerate enthält, die eine mittlere Größe von mindestens 40 µm, insbesondere von mindestens 50 µm besitzen, und/oder dass der Interferenzfarbstoff (22) selbstorganisationsfähige Partikel und/oder Partikelkonglomerate umfasst, die dazu ausgebildet sind, sich in einer geordneten, insbesondere periodischen Struktur anzuordnen.

3. Ophthalmologisches Implantat (10) nach Anspruch 1 oder 2,
**dadurch gekennzeichnet, dass**
der wenigstens eine Interferenzfarbstoff (22) auf dem Grundkörper (12) und/oder innerhalb des Grundkörpers (12) und/oder auf einer Beschichtung des Grundkörpers (12) und/oder innerhalb einer Beschichtung des Grundkörpers (12) und/oder unter einer Beschichtung des Grundkörpers (12) angeordnet ist.

4. Ophthalmologisches Implantat (10) nach einem der Ansprüche 1 bis 3,
**dadurch gekennzeichnet, dass**
die Produktkennzeichnung (18) einen Implantattyp und/oder eine optische Eigenschaft, insbesondere eine Brechkraft, des ophthalmologischen Implantats (10) und/oder einen Datenbankschlüssel und/oder eine Positionierungsinformation charakterisiert.

5. Ophthalmologisches Implantat (10) nach einem der Ansprüche 1 bis 4,
**dadurch gekennzeichnet, dass**
die Produktkennzeichnung (18) wenigstens einen runden und/oder quadratischen und/oder länglichen und/der geometrisch unregelmäßigen Markierungspunkt (20) aus dem wenigstens einen Interferenzfarbstoff (22) umfasst und/oder dass die die Produktkennzeichnung (18) mehrere Markierungspunkte (20) in Form eines linearen Barcodes und/oder eines 2D-Matrixcodes und/oder eines 3D-Matrixcodes und/oder eines Punktrasters mit einem pseudozufälligen unregelmäßigen Charakter umfasst.

6. Ophthalmologisches Implantat (10) nach einem der Ansprüche 1 bis 5,
**dadurch gekennzeichnet, dass**
der wenigstens eine Interferenzfarbstoff (22) mit Licht im Wellenlängenbereich zwischen 300 nm und 3000 nm, insbesondere im Wellenlängenbereich zwischen 400 nm und 750 nm und/oder im Wellenlängenbereich zwischen etwa 750 nm und etwa 3000 nm, wechselwirkt.

7. Ophthalmologisches Implantat (10) nach einem der Ansprüche 1 bis 6,
**dadurch gekennzeichnet, dass**
die Produktkennzeichnung (18) mindestens zwei Interferenzfarbstoffe (22a-f) mit unterschiedlichen Farben umfasst.

8. Ophthalmologisches Implantat (10) nach einem der Ansprüche 1 bis 7,
**dadurch gekennzeichnet, dass**
dieses als Kapselspannring oder als Stent oder als Intraokularlinse oder als implantierbare Kontaktlinse ausgebildet ist.

9. Ophthalmologisches Implantat (10) nach einem der Ansprüche 1 bis 8,
**dadurch gekennzeichnet, dass**
der Grundkörper (12) wenigstens einen optischen Teil (14) und wenigstens einen haptischen Teil (16) umfasst, wobei die Produktkennzeichnung (18) auf dem wenigstens einen optischen Teil (14) und/oder auf dem wenigstens einen haptischen Teil (16) angeordnet ist.

## Claims

1. Ophthalmic implant (10) having a main body (12) which comprises product identification (18) that is preferably machine-readable, wherein the product identification (18) consists at least in part of at least one interference dye (22),
**characterized in that**
the at least one interference dye (22) consists at least in part of nanocellulose, in particular cellulose nanocrystals, cellulose nanofibres, nanofibrillated cellulose and/or bacterial nanocellulose.

2. Ophthalmic implant (10) according to Claim 1,
**characterized in that**
the at least one interference dye (22) contains particles and/or particle agglomerates having a mean dimension of at least 40 µm, in particular of at least 50 µm, and/or in that the interference dye (22) comprises self-organizing particles and/or particle agglomerates which are designed to arrange themselves in an ordered structure, in particular a periodic structure.

3. Ophthalmic implant (10) according to Claim 1 or 2,
**characterized in that**
the at least one interference dye (22) is arranged on the main body (12) and/or within the main body (12) and/or on a coating of the main body (12) and/or within a coating of the main body (12) and/or under a coating of the main body (12).

4. Ophthalmic implant (10) according to any of Claims 1 to 3,
**characterized in that**
the product identification (18) characterizes an implant type and/or an optical property of the ophthalmic implant (10), in particular a refractive power, and/or a database key and/or positioning information.

5. Ophthalmic implant (10) according to any of Claims 1 to 4,
**characterized in that**
the product identification (18) comprises at least one round and/or square and/or elongate and/or geometrically irregular marking point (20) made of the at least one interference dye (22), and/or **in that** the product identification (18) comprises multiple marking points (20) in the form of a linear barcode and/or a 2D matrix code and/or a 3D matrix code and/or a dot grid with a pseudo-random irregular character.

6. Ophthalmic implant (10) according to any of Claims 1 to 5,
**characterized in that**
the at least one interference dye (22) interacts with light in the wavelength range between 300 nm and 3000 nm, in particular in the wavelength range between 400 nm and 750 nm and/or in the wavelength range between about 750 nm and about 3000 nm.

7. Ophthalmic implant (10) according to any of Claims 1 to 6,
**characterized in that**
the product identification (18) comprises at least two interference dyes (22a-f) of different colours.

8. Ophthalmic implant (10) according to any of Claims 1 to 7,
**characterized in that**
the latter is designed as a capsular tension ring or as a stent or as an intraocular lens or as an implantable contact lens.

9. Ophthalmic implant (10) according to any of Claims 1 to 8,
**characterized in that**
the main body (12) comprises at least one optic portion (14) and at least one haptic portion (16), wherein the product identification (18) is arranged on the at least one optic portion (14) and/or on the at least one haptic portion (16).

## Revendications

1. Implant ophtalmologique (10) comprenant un corps de base (12) qui comprend un marquage de produit (18) de préférence lisible par machine, le marquage de produit (18) étant constitué au moins en partie d'au moins un colorant d'interférence (22),
**caractérisé en ce que**
l'au moins un colorant d'interférence (22) est constitué au moins en partie de nanocellulose, notamment de nanocristaux de cellulose, de nanofibres de cellulose, de cellulose nanofibrillée et/ou de nanocellulose bactérienne.

2. Implant ophtalmologique (10) selon la revendication 1,
**caractérisé en ce que**
l'au moins un colorant d'interférence (22) contient des particules et/ou des conglomérats de particules ayant une taille moyenne d'au moins 40 µm, notamment d'au moins 50 µm, et/ou **en ce que** le colorant d'interférence (22) comprend des particules et/ou des conglomérats de particules capables d'auto-organisation, qui sont conçus pour s'agencer en une structure ordonnée, notamment périodique.

3. Implant ophtalmologique (10) selon la revendication 1 ou 2,
**caractérisé en ce que**
l'au moins un colorant d'interférence (22) est agencé sur le corps de base (12) et/ou à l'intérieur du corps de base (12) et/ou sur un revêtement du corps de base (12) et/ou à l'intérieur d'un revêtement du corps de base (12) et/ou sous un revêtement du corps de base (12).

4. Implant ophtalmologique (10) selon l'une des revendications 1 à 3,
**caractérisé en ce que**
le marquage de produit (18) caractérise un type d'implant et/ou une propriété optique, notamment une puissance de réfraction, de l'implant ophtalmologique (10) et/ou une clé de base de données et/ou une information de positionnement.

5. Implant ophtalmologique (10) selon l'une des revendications 1 à 4,
**caractérisé en ce que**
le marquage de produit (18) comprend au moins un point de marquage rond et/ou carré et/ou allongé et/ou de forme géométrique irrégulière (20) composé de l'au moins un colorant d'interférence (22) et/ou **en ce que** le marquage de produit (18) comprend plusieurs points de marquage (20) sous la forme d'un code-barres linéaire et/ou d'un code matriciel 2D et/ou d'un code matriciel 3D et/ou d'une trame de points présentant un caractère pseudo-aléatoire irrégulier.

6. Implant ophtalmologique (10) selon l'une des revendications 1 à 5,
**caractérisé en ce que**
l'au moins un colorant d'interférence (22) interagit avec la lumière dans la plage de longueurs d'onde comprise entre 300 nm et 3 000 nm, notamment dans la plage de longueurs d'onde comprise entre 400 nm et 750 nm et/ou dans la plage de longueurs d'onde comprise entre environ 750 nm et environ 3 000 nm.

7. Implant ophtalmologique (10) selon l'une des revendications 1 à 6,
**caractérisé en ce que**
le marquage de produit (18) comprend au moins deux colorants d'interférence (22a-f) de couleurs différentes.

8. Implant ophtalmologique (10) selon l'une des revendications 1 à 7,
**caractérisé en ce que**
celui-ci est conçu sous forme d'anneau de tension capsulaire ou sous forme de stent ou sous forme de lentille intraoculaire ou sous forme de lentille de contact implantable.

9. Implant ophtalmologique (10) selon l'une des revendications 1 à 8,
**caractérisé en ce que**
le corps de base (12) comprend au moins une partie optique (14) et au moins une partie haptique (16), le marquage du produit (18) étant agencé sur l'au moins une partie optique (14) et/ou sur l'au moins une partie haptique (16).
